# EUROPÄISCHE PATENTSCHRIFT

(11) **EP 1 643 949 B1**
(45) Veröffentlichungstag und Bekanntmachung des Hinweises auf die Patenterteilung: **07.02.2007**
(21) Anmeldenummer: 04803603.2
(22) Anmeldetag: 08.12.2004
(51) Int. Cl.: A61F 13/56, A61F 13/49

(54) **VERFAHREN UND VORRICHTUNG ZUM APPLIZIEREN EINES FLACHMATERIALBAHNABSCHNITTS**
METHOD AND DEVICE FOR APPLYING A FLAT MATERIAL WEB SECTION
PROCEDE ET DISPOSITIF POUR APPLIQUER UN SEGMENT DE BANDE DE MATERIAU PLAT

(30) Priorität: 30.12.2003 DE 10361856
(43) Veröffentlichungstag der Anmeldung: 12.04.2006
(73) Patentinhaber: Paul Hartmann Aktiengesellschaft, 89522 Heidenheim (DE)
(72) Erfinder: LOHRENGEL, Armin, 89522 Heidenheim (DE); FRANK, Bernd, 89555 Steinheim (DE); SPRICK, Ralf, 89522 Heidenheim (DE)
(74) Vertreter: Friz, Oliver
(86) Internationale Anmeldenummer: PCT/EP2004/013922
(87) Internationale Veröffentlichungsnummer: WO 2005/065617

(56) Entgegenhaltungen:
- US-A- 4 767 487

## Beschreibung

Die vorliegende Erfindung betrifft ein Verfahren zum Applizieren von Flachmaterialbahnabschnitten auf eine sich in einer Maschinenrichtung mit einer ersten Bahngeschwindigkeit bewegende erste Flachmaterialbahn im Zuge der Herstellung von Hygieneartikeln oder medizinischen Artikeln, wobei die Abmessung der Flachmaterialbahnabschnitte in der Maschinenrichtung geringer ist als die Abmessung der herzustellenden Artikel, wobei die Flachmaterialbahnabschnitte von einer Endlosbahn im "Cut-and-Place"-Verfahren abgetrennt und auf die erste Flachmaterialbahn aufgebracht werden, und wobei die Endlosbahn mit einer zweiten Bahngeschwindigkeit in Richtung auf eine Messerwalze einer Schneidstation zugeführt wird. Die erste Flachmaterialbahn kann auch eine Aufeinanderfolge von diskreten Artikeln sein. Des Weiteren betrifft die Erfindung eine Vorrichtung zum Durchführen eines solchen Verfahrens, mit einer Messerwalze und einer mit dieser zusammenwirkenden Ambosswalze.

Beispielsweise kann es sich bei den zu applizierenden Flachmaterialbahnabschnitten um Verschlusslaschen in Form von klebenden Tapeabschnitten oder in Form von mechanische Verschlusselemente aufweisenden Abschnitten handeln, oder um Wundauflagen oder Anfasselemente von Verbandmaterialien, insbesondere von Pflastern.

Von der in Maschinenrichtung bewegten ersten Flachmaterialbahn werden die herzustellenden Artikel, z. B. Hygieneartikel, wie Windeln oder Windelhöschen, als Abschnitte der insbesondere endlos zugeführten Flachmaterialbahn abgetrennt. Für eine wirtschaftliche Herstellung der Artikel werden auch die zu applizierenden Flachmaterialbahnabschnitte von einer Endlosbahn im "Cut-and-Place"-Verfahren abgetrennt und auf die sich in der Maschinenrichtung bewegende erste Flachmaterialbahn aufgebracht. Da die Längserstreckung der zu applizierenden Flachmaterialbahnabschnitte sehr viel geringer ist als die Längserstreckung eines herzustellenden Artikels, ist demzufolge die erste Bahngeschwindigkeit der ersten Flachmaterialbahn größer als die zweite Bahngeschwindigkeit, mit der die Endlosbahn zur Bildung der Flachmaterialbahnabschnitte zugeführt wird.

Im Einzelnen treten insbesondere folgende Probleme auf:

Wenn die mit geringerer Geschwindigkeit zugeführte Endlosbahn dauerhaft schlupfbehaftet an eine Ambosswalze anliegt, so führt dies zu abrasivem Verschleiß der Endlosbahn. In diesem Kontaktbereich ist die Umfangsgeschwindigkeit der Ambosswalze um ein Vielfaches, oftmals um über das 30-Fache höher als die Geschwindigkeit der Endlosbahn. Durch entstandenen Abrieb bei der Endlosbahn wird die Herstellungsvorrichtung verschmutzt und Schmutzpartikel werden außerdem auf die herzustellenden Artikel übertragen.

Insbesondere wenn mit klebenden Beschichtungen bei der Endlosbahn gearbeitet wird, gelangen beim Quetschschnitt gebildete oder abgescherte Kleberanteile auf die Ambosswalze und werden dort durch den Schlupf der Endlosbahn am gesamten Umfang der Ambosswalze verteilt. Sie werden auch mit den abzutrennenden Flachmaterialbahnabschnitten auf den herzustellenden Artikel transportiert.

Wenn Flachmaterialbahnabschnitte von der Endlosbahn bei verhältnismäßig geringen Geschwindigkeiten abgelängt werden und dann in der Folge auf höhere Geschwindigkeiten beschleunigt werden zum Applizieren auf die erste Flachmaterialbahn, ergibt sich das Problem, dass träge Massen mit hoher Frequenz beschleunigt und wieder verzögert werden müssen. Wenn mit verhältnismäßig biegesteifen Materialien für die Bildung der Flachmaterialbahnabschnitte, insbesondere mit gefalteten und demnach mehrfach übereinander liegenden Materialien gearbeitet wird, so ist es problematisch, zur Reduzierung von bewegten Massen mit verringerten Walzendurchmessern zu arbeiten, da sich solchenfalls die biegesteifen Materialien und hiervon abgetrennte Flachmaterialbahnabschnitte kaum an der Oberfläche der Ambosswalze oder an sonstigen Transportwalzen halten lassen. Dies würde aber zu Störungen im Produktionsablauf führen, die unter wirtschaftlichen Gesichtspunkten nicht hingenommen werden können.

Insofern war es seither nicht möglich, die vorstehend genannten Probleme gleichermaßen befriedigend zu lösen.

Der vorliegenden Erfindung liegt die Aufgabe zugrunde, ein Verfahren und eine Vorrichtung zur Durchführung des Verfahrens zu schaffen, die es gestatten, Flachmaterialbahnabschnitte auf eine in einer Maschinenrichtung laufende erste Flachmaterialbahn zu applizieren, wobei eine wirtschaftliche Herstellbarkeit mit hoher Prozessstabilität geschaffen werden soll, die von dem einen oder anderen vorstehend genannten Problem unbeeinflusst bleibt.

Diese Aufgabe wird bei einem Verfahren der genannten Art erfindungsgemäß dadurch gelöst, dass ein vorderer Abschnitt der Endlosbahn gegen einen Oberflächenabschnitt einer mit der Messerwalze zusammenwirkenden Ambosswalze angelegt wird, der weniger gekrümmt ist als der Umkreis der Ambosswalze, und dass der vordere Abschnitt zur Bildung des Flachmaterialbahnabschnitts von der Endlosbahn abgelängt wird und ein jeweiliger abgelängter Flachmaterialbahnabschnitt schlupffrei durch die Ambosswalze beschleunigt wird.

Nach dem erfindungsgemäßen Verfahren wird ein vorderer Abschnitt der Endlosbahn, der dann unmittelbar zur Bildung des zu applizierenden Flachmaterialbahnabschnitts von der Endlosbahn abgetrennt wird, nur verhältnismäßig wenig gekrümmt, indem er an den vorgesehen Oberflächenbereich der Ambosswalze angelegt wird. Dieser Oberflächenbereich ist nämlich so gestaltet, dass er weniger gekrümmt ist als ein sich theoretisch ergebender zylindrischer Umfang einer zylindrischen Ambosswalze. Die Krümmung dieses Oberflächenabschnitts der Ambosswalze und damit die Krümmung des hiergegen angelegten Abschnitts der Endlosbahn ist erfindungsgemäß geringer als die Krümmung eines Umkreises der Ambosswalze. Unter dem Umkreis der Ambosswalze wird vorliegend der kreisförmige Querschnitt oder der Querschnitt einer theoretischen zylindrischen Mantelfläche verstanden, den der von der Drehachse der Ambosswalze entfernteste Punkt des besagten Oberflächenbereichs der Ambosswalze beschreibt.

Wenn nach einer Ausführungsform der Erfindung der besagte Oberflächenabschnitt der Ambosswalze im Schnitt betrachtet einer Kreisform folgt, so ist der Krümmungsradius dieser Kreisform größer als der Radius des Umkreises der Ambosswalze. Der Oberflächenabschnitt der Ambosswalze muss aber nicht notwendigerweise kreisförmig gekrümmt sein (dies stellt nur einen Spezialfall einer gekrümmten Oberfläche dar); er kann beispielsweise einer beliebigen Polynomkurvenform folgen oder ebene Flächenabschnitte, die eine Polygonform bilden, umfassen. Es wäre auch denkbar dass der Oberflächenabschnitt einen unendlichen Krümmungsradius aufweist, also im Grenzfall eben ist.

Die Erfindung erweist sich in mehrfacher Hinsicht als besonders vorteilhaft. Zum einen wird ermöglicht, dass der zu applizierende Flachmaterialbahnabschnitt bei einer betrachteten Größe oder bei einem betrachteten Durchmesser der Ambosswalze weniger stark gekrümmt werden muss. Anders ausgedrückt kann bei einer vorgegebenen nicht zu unterschreitenden Krümmung des Flachmaterialbahnabschnitts mit geringeren Durchmessern der Ambosswalze gearbeitet werden. Letzteres hat aber zur Folge, dass die Masse der Ambosswalze geringer gehalten werden kann, was wiederum die Realisierung dynamischer Beschleunigungs- und Verzögerungsvorgänge begünstigt.

In bevorzugter Weiterbildung der Erfindung wird der Flachmaterialbahnabschnitt nach dem Abtrennen von der Endlosbahn derart beschleunigt, dass er im wesentlichen mit der ersten Bahngeschwindigkeit auf die erste Flachmaterialbahn appliziert wird. Dieser Beschleunigungsvorgang kann durch die erwähnte Ambosswalze allein oder durch eine oder mehrere daran anschließende Transportwalzen, an die der jeweilige Flachmaterialbahnabschnitt übergeben wird, ausgeführt werden.

Es erweist sich als vorteilhaft, wenn der vordere Abschnitt der Endlosbahn, der dann nach dem Abtrennen von der Endlosbahn den Flachmaterialbahnabschnitt bildet, gegen den Oberflächenabschnitt der Ambosswalze durch Unterdruck angesaugt wird.

Wie bereits vorausgehend erwähnt, erweist es sich als vorteilhaft, wenn die Winkelgeschwindigkeit der Ambosswalze periodisch veränderlich gesteuert wird.

Im Zusammenwirken mit der Messerwalze bei der Aufnahme des vorderen Abschnitts der Endlosbahn erweist es sich als vorteilhaft, wenn die Winkelgeschwindigkeit der Ambosswalze niedrig ist und vorzugsweise im Wesentlichen der Zuführgeschwindigkeit der Endlosbahn, also der zweiten Bahngeschwindigkeit entspricht. Vorzugsweise im Wesentlichen unmittelbar nach dem Ablängen des Flachmaterialbahnabschnitts von der Endlosbahn wird die Ambosswalze zusammen mit dem Flachmaterialbahnabschnitt stark beschleunigt. Es ist denkbar und vorteilhaft, dass die Ambosswalze den Flachmaterialbahnabschnitt auf eine Umfangs- bzw. Bahngeschwindigkeit beschleunigt, die im Wesentlichen der ersten Bahngeschwindigkeit entspricht. Es wäre aber auch denkbar, dass eine weitere Transportwalze oder mehrere Transportwalzen vorgesehen sind, die die schlussendliche Beschleunigung des Flachmaterialbahnabschnitts auf die schlussendliche erste Bahngeschwindigkeit ausführen. Des Weiteren sei erwähnt, dass beim Applizieren des Flachmaterialbahnabschnitts auf die erste Flachmaterialbahn die Verwendung einer Gegendruckwalze im Zusammenwirken mit einer Applikationswalze, die direkt von der Ambosswalze gebildet sein kann, empfohlen wird. Die Andruckwalze rollt auf der von der Applikationswalze abgewandten Seite der Flachmaterialbahn gegen die Oberfläche der Flachmaterialbahn ab und bildet ein Gegenlager beim Applizieren des Flachmaterialbahnabschnitts auf die erste Flachmaterialbahn.

Da es sich als vorteilhaft erweist, dass die Oberflächengeschwindigkeit der Applikationswalze, insbesondere der Ambosswalze und damit die Geschwindigkeit des Flachmaterialbahnabschnitts im Wesentlichen der Bahngeschwindigkeit der ersten Flachmaterialbahn beim Applizieren entspricht, wird auf folgendes hingewiesen: Aufgrund der erfindungsgemäß geringen Krümmung des Oberflächenabschnitts der Ambosswalze und damit der Krümmung des Flachmaterialbahnabschnitts, die beide von der Krümmung des theoretischen Umkreises der Ambosswalze abweichen, muss die Winkelgeschwindigkeit der Ambosswalze beim Applizieren periodisch verändert werden, damit die Bahngeschwindigkeit des Flachmaterialbahnabschnitts vorzugsweise über die gesamte Phase des Applizierens konstant ist und vorzugsweise im Wesentlichen der ersten Bahngeschwindigkeit entspricht. Die im Einzelfall konkret zu verfolgende periodische Steuerung der Winkelgeschwindigkeit der Ambosswalze ergibt sich aus der konkret verwandten Geometrie des Oberflächenabschnitts, gegen den der Flachmaterialbahnabschnitt angelegt wird.

In vorteilhafter Weiterbildung des Erfindungsgedankens wird vorgeschlagen, dass die Winkelgeschwindigkeit der Ambosswalze während des Aufnehmens und Ablängens des Flachmaterialbahnabschnitts derart gesteuert wird, dass die sich hieraus ergebende Geschwindigkeit des Flachmaterialbahnabschnitts der zweiten Bahngeschwindigkeit, also der Zuführgeschwindigkeit der Endlosbahn, entspricht. Aufgrund der von einer Kreisform um den Drehmittelpunkt der Ambosswalze abweichenden Geometrie des Oberflächenabschnitts erfordert dies wiederum periodische Steuervorgänge der Rotationsgeschwindigkeit der Ambosswalze.

Die vorliegende Erfindung erweist sich in Verbindung mit Flachmaterialbahnabschnitten bzw. Endlosbahnen als besonders vorteilhaft, die um eine in ihrer Längsrichtung verlaufende Achse oder um mehrere in ihrer Längsrichtung verlaufende Achsen gefaltet, insbesondere Z-förmig gefaltet sind. Durch eine solche Z-Faltung, wie sie vorteilhafterweise beim Applizieren von Verschlusselementen für wegwerfbare absorbierende Hygieneartikel Verwendung finden kann, erweist sich die vorliegende Erfindung als besonders vorteilhaft, weil solche einfach oder mehrfach auf sich selbst gefaltete Endlosbahnen oder Flachmaterialbahnabschnitte eine größere Biegesteifigkeit aufweisen als Einfachmaterialien. Sie lassen sich deshalb sehr viel schwerer gegen gekrümmte Oberflächen anlegen, insbesondere für Transportzwecke in schnelllaufenden Herstellungsanlagen fixieren, insbesondere ansaugen. Wenn solche einfach oder mehrfach gefalteten Flachmaterialbahnabschnitte appliziert werden und im Zuge dessen zu stark gekrümmt werden, so lösen sich gegebenenfalls vorgesehene Schweiß-, Klebe- oder Perforationsstellen, welche die aufeinandergefalteten Bahnabschnitte lösbar aneinander halten. Dies führt zu Störungen im Produktionsablauf. Die aufeinander gefalteten Bahnabschnitte sollen möglichst bis zur Ingebrauchnahme durch die genannten Mittel in Anlage aneinander gehalten werden.

Die eingangs genannte Aufgabe wird aber auch durch eine Vorrichtung mit den Merkmalen des Anspruchs 11 gelöst. Diese erfindungsgemäße Vorrichtung ist dadurch gekennzeichnet, dass die Ambosswalze einen mit dem abzutrennenden Flachmaterialbahnabschnitt zusammenwirkenden Oberflächenabschnitt aufweist, der weniger gekrümmt ist als der Umkreis der Ambosswalze. Der Begriff des Umkreises der Ambosswalze ist wiederum im eingangs geschilderten Sinn definiert.

Insbesondere kann der Oberflächenabschnitt zylindrisch gekrümmt sein. Solchenfalls ist sein Krümmungsradius größer als der Krümmungsradius des Umkreises der Ambosswalze. Der Krümmungsradius des Oberflächenabschnitts ist vorzugsweise 50 -250 mm, insbesondere 65 - 200 mm, insbesondere 80 - 150 mm, insbesondere 90 - 120 mm.

Der Krümmungsradius des Oberflächenabschnitts beträgt in Weiterbildung der Erfindung wenigstens das 1,5-fache, insbesondere wenigstens das 1,7-fache, insbesondere wenigstens das 1,8-fache, insbesondere wenigstens das 1,9-fache, insbesondere wenigstens das 2-fache des Radius des Umkreises der Ambosswalze.

Nach einer vorteilhaften Ausführungsform der erfindungsgemäßen Vorrichtung beträgt der Radius des Umkreises der Ambosswalze 25 - 75 mm, insbesondere 35 - 65 mm, insbesondere 42 - 52 mm.

Die Messerwalze trägt an ihrem Umfang wenigstens zwei, vorzugsweise wenigstens drei Messer. Diese Messer können in vorteilhafterweise an der Messerwalze federnd nachgiebig gehalten sein. Eine solche federnde Anordnung der Messer kann in an sich beliebiger Weise ausgeführt sein. Nach einer bevorzugten Ausführung umfasst die Walze einen bogenförmig, insbesondere U- oder S-förmig gekrümmten Haltearm, an dem die Messer starr, also nicht federnd, montierbar sind, wobei der Haltearm selbst eine federnde radiale Nachgiebigkeit aufweist.

Es erweist sich des Weiteren als besonders vorteilhaft, wenn die Ambosswalze so massearm wie möglich ausgebildet ist. Dies lässt sich beispielsweise dadurch erreichen, dass die Walze in Umfangsrichtung zwischen Oberflächenabschnitten für die Anlage von zu applizierenden Flachmaterialbahnabschnitten in radialer Richtung zurückgesetzt ist, vorteilhafterweise bis wenigstens nahezu an einen inneren Nabenabschnitt der Ambosswalze. Es hat sich als zweckmäßig erwiesen, wenn die Ambosswalze einen einzigen Oberflächenabschnitt zur Aufnahme des Flachmaterialbahnabschnitts aufweist, der etwa 45° bis 120°, insbesondere 80° bis 110° in Umfangsrichtung umspannt. Wie vorausgehend bereits angedeutet wurde, erweist es sich als vorteilhaft, wenn die erfindungsgemäße Vorrichtung eine Antriebssteuereinrichtung aufweist, mittels derer die Winkelgeschwindigkeit der Messerwalze und der Ambosswalze periodisch veränderbar sind. Die Steuerparameter werden dabei in Abhängigkeit von der Geometrie der Walzen und der Oberflächenbereiche zur Aufnahme der Flachmaterialbahnabschnitte so bestimmt, dass erwünschte Geschwindigkeiten bei der Übernahme eines Flachmaterialbahnabschnitts und beim Applizieren des Flachmaterialbahnabschnitts auf die erste Flachmaterialbahn reproduzierbar erreicht werden.

Insbesondere ist die erfindungsgemäße Vorrichtung so ausgebildet, dass die erste Bahngeschwindigkeit mindestens 50 m/min und bis zu 400 m/min beträgt.

Die zweite Bahngeschwindigkeit, mit der die Endlosbahn für die Bildung der zu applizierenden Flachmaterialbahnabschnitte zugeführt wird, hängt von der Abschnittslänge des zu applizierenden Flachmaterialbahnabschnitts im Verhältnis zur Länge des herzustellenden Artikels ab und beträgt vorzugsweise 5 bis 80 m/min.

Bevorzugte Abmessungen der herzustellenden Artikel und der zu applizierenden Flachmaterialbahnabschnitte ergeben sich aus den weiteren Ansprüchen.

Weitere Merkmale, Einzelheiten und Vorteile der Erfindung ergeben sich aus den beigefügten Patentansprüchen und aus der zeichnerischen Darstellung und nachfolgenden Beschreibung des erfindungsgemäßen Verfahrens bzw. einer schematischen Darstellung der erfindungsgemäßen Herstellungsvorrichtung sowie eines herzustellenden Artikels.

In der Zeichnung zeigt:
- Figur 1: eine schematische Darstellung einer erfindungsgemäßen Vorrichtung zum Applizieren von Flachmaterialbahnabschnitten auf eine schnelllaufende Flachmaterialbahn;
- Figur 2: eine schematische Darstellung einer Messerwalze, einer Ambosswalze und einer Andruckwalze der erfindungsgemäßen Vorrichtung;
- Figur 3: eine Schnittansicht der erfindungsgemäß ausgebildeten Ambosswalze;
- Figur 4: eine perspektivische Ansicht einer von einer Rolle abrollbaren Endlosbahn zur Bildung von zu applizierenden Flachmaterialbahnabschnitten;
- Figur 5: eine perspektivische Ansicht einer schraubenförmig aufgerollten Endlosbahn;
- Figur 6: eine Draufsicht auf einen zu applizierenden Flachmaterialbahnabschnitt in Form einer Verschlusslasche für einen absorbierenden Hygieneartikel;
- Figur 7: eine Schnittansicht des Flachmaterialbahnabschnitts nach Figur 2;
- Figur 8: eine schematische Ansicht des Flachmaterialbahnabschnitts nach Figuren 6 und 7 in Z-förmig gefalteter Konfiguration und
- Figur 9: eine Draufsicht auf einen Hygieneartikel mit einer Verschlusslasche nach Figuren 6 bis 8.

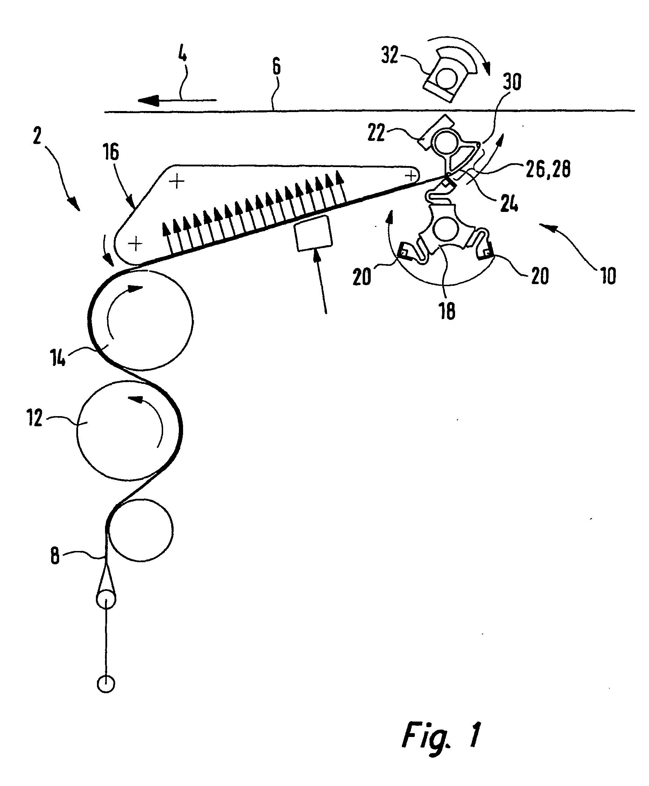

Figur 1 zeigt eine insgesamt mit dem Bezugszeichen 2 bezeichnete Vorrichtung zum Applizieren eines noch näher zu beschreibenden Flachmaterialbahnabschnitts auf eine sich in einer Maschinenrichtung 4 kontinuierlich und mit hoher Geschwindigkeit bewegende erste Flachmaterialbahn 6. Bei dieser ersten Flachmaterialbahn 6 kann es sich beispielsweise um eine Bahn zur Herstellung absorbierender Hygieneartikel handeln, die zur Vereinzelung als Längsabschnitte von der ersten Flachmaterialbahn abgetrennt werden. Es kann sich aber auch um medizinische Artikel, wie Verbandmaterialien, insbesondere Pflaster, handeln.

Des Weiteren zeigt Figur 1 eine schematisch angedeutete Endlosbahn 8, die von einer nicht dargestellten Rolle abgerollt und einer insgesamt mit dem Bezugszeichen 10 bezeichneten Schneidstation mit einer zweiten Bahngeschwindigkeit zugeführt wird. Diese Zuführung wird über einen sogenannten Umschlingungsantrieb in Form zweier Transportwalzen 12 und 14 und einen Saugbandantrieb 16 ausgeführt. Die Endlosbahn 8 wird dabei schlupffrei in Richtung auf die Schneidstation 10 zugeführt.

Die Schneidstation 10 umfasst eine Messerwalze 18 mit wenigstens zwei, im dargestellten Fall drei Schneidmessern 20. Ferner vorgesehen ist eine Ambosswalze 22 mit einem Oberflächenabschnitt 24, der mit einem vorderen Abschnitt 26 der Endlosbahn 8 und mit den Schneidmessern 20 zusammenwirkt. Im Zuge der Zuführung der Endlosbahn 8 wird der vordere Abschnitt 26 der Endlosbahn 8 in die Schneidstation 10 hineingefördert. Dabei wird der vordere Abschnitt 26 gegen den Oberflächenabschnitt 24 der Ambosswalze 22 angelegt und durch Unterdruckbeaufschlagung an den Oberflächenabschnitt 24 angesaugt. Im Zuge des gegensinnigen Antriebs der Messerwalze 18 und der Ambosswalze 24 führt das in Rotationsrichtung nachfolgende Messer 20 eine Ablängung des vorderen Abschnitts 26 von der Endlosbahn 8 aus, so dass der gegen den Oberflächenabschnitt 24 anliegende vordere Abschnitt 26 einen abgetrennten Flachmaterialbahnabschnitt 28 bildet, der durch die Ambosswalze 42 unmittelbar nachfolgend sehr stark beschleunigt wird. Im dargestellten Fall beschleunigt die Ambosswalze 22 den Flachmaterialbahnabschnitt 28 von im wesentlichen der zweiten Bahngeschwindigkeit der Endlosbahn 8 auf vorzugsweise im Wesentlichen die erste Bahngeschwindigkeit der ersten Flachmaterialbahn 6. Betrachtet man die Darstellung der Figur 1, so wird nach einer bevorzugten Ausführungsform der Erfindung die Ambosswalze 22 derart periodisch beschleunigt und verzögert, dass das in Rotationsrichtung vorne liegende Ende 30 des Flachmaterialbahnabschnitts 28 bei Erreichen der "12-Uhr-Stellung" die erste Bahngeschwindigkeit der ersten Flachmaterialbahn 6 erreicht haben wird. In weiterer Ausbildung der Erfindung wird die Winkelgeschwindigkeit der Ambosswalze 22 derart gesteuert, dass während des gesamten Applikationsvorgangs des Flachmaterialbahnabschnitts 28 auf die erste Flachmaterialbahn 6 die Bahngeschwindigkeit des Flachmaterialbahnabschnitts 28 vorzugsweise durchgehend im Wesentlichen der ersten Bahngeschwindigkeit entspricht.

Während des Applizierens des Flachmaterialbahnabschnitts 28 auf die erste Flachmaterialbahn wirkt die Ambosswalze 22 mit einer Andrückwalze 32 zusammen, die auf der der Ambosswalze 22 abgewandten Seite der ersten Flachmaterialbahn 6 vorgesehen ist und gegensinnig zur Ambosswalze 22, jedoch vorzugsweise synchron zu dieser angetrieben wird.

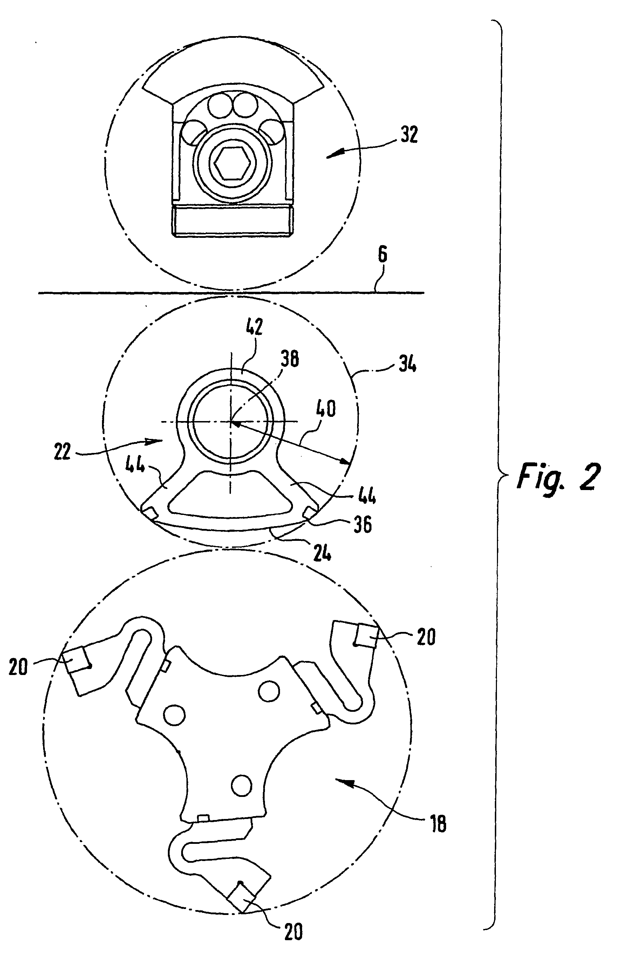

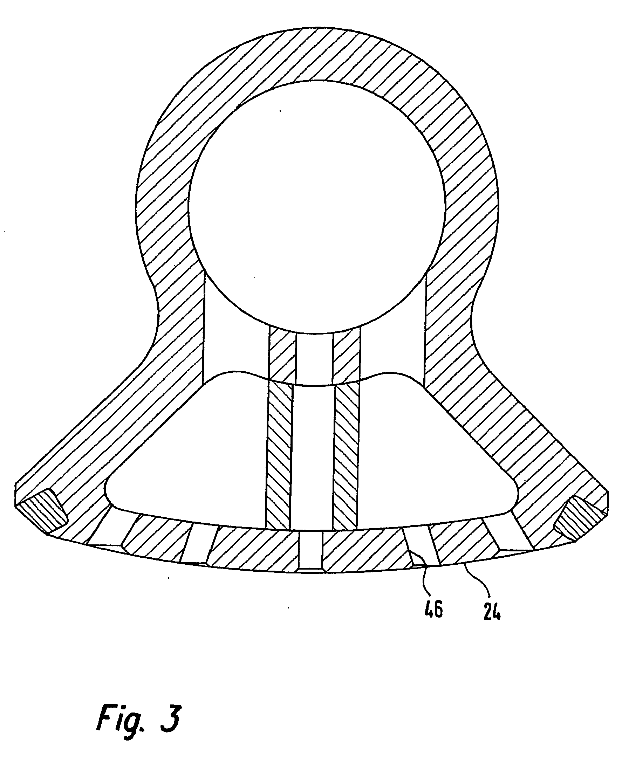

Figur 2 zeigt die Messerwalze 18, die Ambosswalze 22 und die Andrückwalze 32. Man erkennt, dass die Krümmung des Oberflächenabschnitts 24 der Ambosswalze sehr viel geringer ist als die Krümmung des Umkreises 34 der Ambosswalze 22, der gebildet ist aus der Kreisbahn eines radial äußeren Punkts 36 des Oberflächenabschnitts 24 bei seiner Bewegung um den Rotationspunkt 38 der Ambosswalze 22. Wenn der Oberflächenabschnitt 24 einen Abschnitt einer Zylindermantelfläche bildet, also im Querschnitt eine Kreisbahn beschreibt, so ist deren Krümmungsradius sehr viel größer als der Krümmungsradius 40 des Umkreises 34. Auf diese Weise wird erfindungsgemäß erreicht, dass ein gegen den Oberflächenabschnitt 24 angelegter, vorzugsweise insbesondere angesaugter vorderer Abschnitt 26 der Endlosbahn 8 weit weniger stark gekrümmt wird, als dies der Fall wäre, wenn die Ambosswalze 22 einen zylinderförmigen Mantel aufwiese. Es erweist sich als vorteilhaft, dass trotz der geringen Krümmung, insbesondere trotz des sehr großen Krümmungsradius des Oberflächenabschnitts 24 eine verhältnismäßig kompakte Ausbildung der Ambosswalze 22 mit einem Radius des Umkreises 34 von vorzugsweise 25 bis 75 mm gewählt werden kann. Die Masse der Ambosswalze 22 und damit die träge Masse, die bei dynamischen Beschleunigungs- und Verzögerungsvorgängen überwunden werden muss, ist damit beherrschbar gering. Hinzu kommt, dass, wie die Figur 2 zeigt, die Ambosswalze 22 mit Ausnahme des den Oberflächenabschnitt 24 bildenden Bereichs in radialer Richtung nach innen zurückgesetzt ist, was mit einer weiteren Massereduzierung verbunden ist. Die Ambosswalze 22 besteht im Wesentlichen aus einem eine Antriebswelle umschließenden Nabenbereich 42 und zwei hiervon ausgehenden und im Wesentlichen in radialer Richtung nach außen erstreckten Streben 44, welche den Oberflächenabschnitt 24 tragen. Aus der Detaildarstellung der Figur 3 erkennt man in dem Oberflächenabschnitt 24 mündende Öffnungen 46, die von radial innen mit Unterdruck beaufschlagbar sind und den vorderen Abschnitt 26 bzw. den Flachmaterialbahnabschnitt 28 gegen den Oberflächenabschnitt 24 ansaugen.

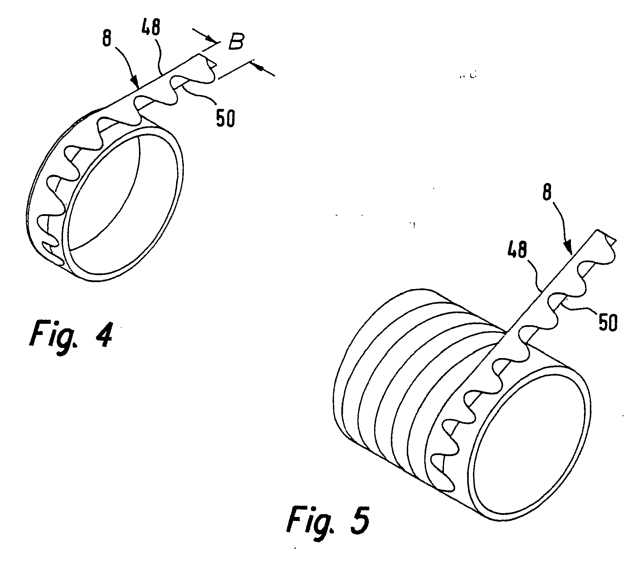

Die Figuren 4 und 5 zeigen perspektivisch die Endlosbahn 8, die von einer Rolle abrollbar und der erfindungsgemäßen Vorrichtung 2 nach Figur 1 zuführbar ist. Die Wicklung der Figur 5 ist schrauben- oder wendelförmig. Man erkennt, dass die Endlosbahn 8 um eine in Längsrichtung verlaufende Achse 48 gefaltet ist. Die Faltung kann aber auch Z-förmig sein, und um eine weitere in Längsrichtung verlaufende Achse 50 umgeschlagen sein.

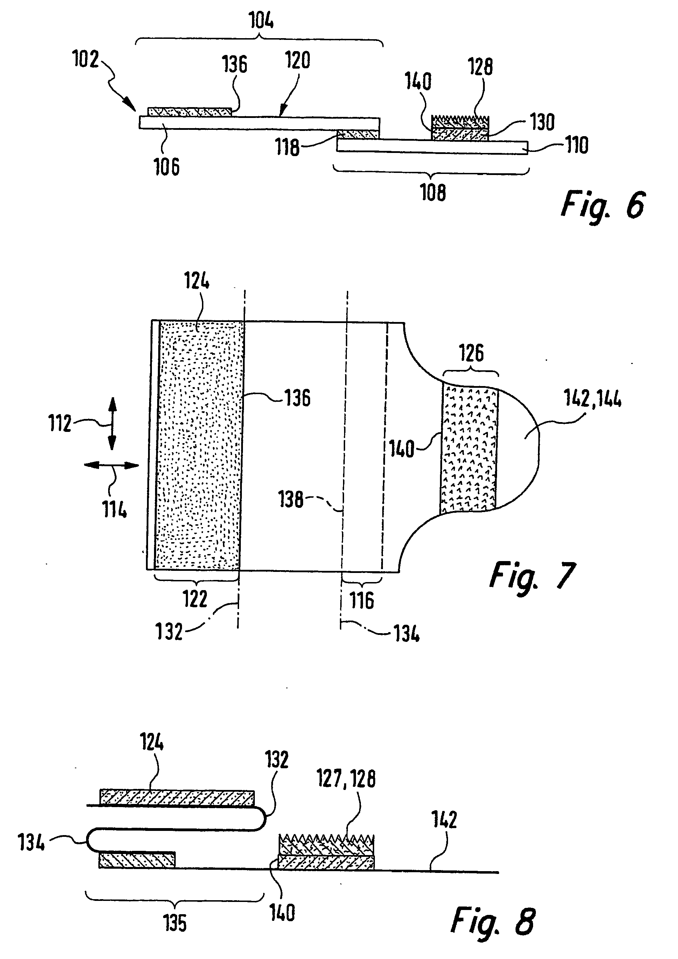

Die Figuren 6 bis 8 zeigen ein Befestigungselement, welches insgesamt mit dem Bezugszeichen 102 bezeichnet ist. Das Befestigungselement 102 umfaßt einen ersten Abschnitt 104 einer ersten Trägerschicht 106 und einen zweiten Abschnitt 108 einer zweiten Trägerschicht 110.

Das Befestigungselement 102 ist als Flachmaterialbahnabschnitt 28 von der Endlosbahn 8 abgetrennt worden, wobei sich die Endlosbahn 8 in der in Figur 7 angedeuteten Maschinenrichtung 112 erstreckt. Der zweite Abschnitt 108 ist in einer Querrichtung 114 neben dem ersten Abschnitt 104 angeordnet, wobei im dargestellten Fall der erste Abschnitt 104 und der zweite Abschnitt 108 einander überlappen, so dass ein Überlappungsbereich 116 gebildet ist, an dem die beiden Abschnitte 104, 108 über einen Kleber 118, über Schweißstellen oder in sonstiger Weise unlösbar miteinander verbunden sind.

Auf einer ersten Oberseite 120 des ersten Abschnitts 104 ist ein erster Bereich 122 vorgesehen, der eine Kleberbeschichtung 24 trägt, mit dem das Befestigungselement an einen Hygieneartikel angefügt werden kann.

In einem zweiten Bereich 126 des zweiten Abschnitts 108 sind Fixiermittel 127 in Form von mechanisch wirkenden Verschlusselementen 128, vorzugsweise in Form einer hakenbildenden Komponente eines Haken/Schlaufen-Materials, vorgesehen, insbesondere mittels eines Klebers 130 aufgeklebt. Der zweite Bereich 126 ist vorzugsweise auf derselben Oberseite 120 des Befestigungselements 102 vorgesehen wie der erste Bereich 122.

Des weiteren sind in Figur 7 erste und zweite Falzlinien 132, 134 (die den Falzlinien 48, 50 der Figuren 4 und 5 entsprechen) angedeutet und aus Figur 8 ersichtlich, um welche das Befestigungselement 102 in Längsrichtung 112 der Endlosbahn 8 Z-förmig gefaltet ist, so dass die in Figur 8 dargestellte Konfiguration 135 gebildet wird. In vorteilhafter Weise verläuft die erste Falzlinie 132 unmittelbar entlang eines Längsrands 136 der Kleberbeschichtung 124 im ersten Bereich 122. Die zweite Falzlinie 134 läuft vorteilhafterweise unmittelbar entlang des Materialübergangs zwischen erstem und zweitem Abschnitt 104 bzw. 108, also entlang eines Rands 138 des Überlappungsbereichs 116.

Wie aus Figur 8 ersichtlich ist der zweite Bereich 126 des zweiten Abschnitts 108 in Querrichtung 114 derart weit vom Überlappungsbereich 116 oder von einem sonstigen Materialübergangsbereich zwischen erstem Abschnitt 104 und zweitem Abschnitt 108 beabstandet, dass er sich in Querrichtung 114 außerhalb der Z-förmig gefalteten Konfiguration 135 des Befestigungselements 102 befindet, was sich in mehrfacher Hinsicht als vorteilhaft erweisen kann. Im dargestellten Fall reicht ein dem ersten Abschnitt 104 zugewandter Längsrand 140 des zweiten Bereichs 126 und damit der mechanisch wirkenden Verschlußelemente 128 in Querrichtung 114 nahezu an die erste Falzlinie 136 bei Z-förmig gefalteter Konfiguration 125 heran. Es wäre aber auch denkbar, dass der zweite Bereich 126 so bezüglich des zweiten Abschnitts 108 positioniert ist, dass die mechanisch wirkenden Verschlusselemente 128 bzw. deren Längsrand 140 einen Abstand von einigen Millimetern von der Z-förmig gefalteten Konfiguration 135 des Befestigungselements aufweisen.

Es sei noch erwähnt, dass der erste Abschnitt 104 in Querrichtung 114 elastisch dehnbar ausgebildet ist und dass der zweite Abschnitt 108 in Querrichtung 114 im wesentlichen nicht dehnbar ausgebildet ist.

Eine Kleberbeschichtung des ersten Bereichs 124 sowie das Fixiermittel 127 im zweiten Bereich 126 können in Längsrichtung 112 durchgehend und endlos auf eine dementsprechende Endlosbahn 8 aus der ersten Trägerschicht 106 und der zweiten Trägerschicht 108 aufgebracht werden. In entsprechender Weise werden die erste und die zweite Trägerschicht 106, 108 durch einen endlosen Kleberstreifen 118 oder in sonstiger Weise in Längsrichtung 112 kontinuierlich miteinander verbunden. Eine Simultanfertigung zweier spiegelbildlich jedoch in Längsrichtung um n/2 versetzter Endlosbahnen 8, wie dies beispielsweise aus EP 0 669 121 A1 bekannt ist, ist ebenfalls denkbar und vorteilhaft.

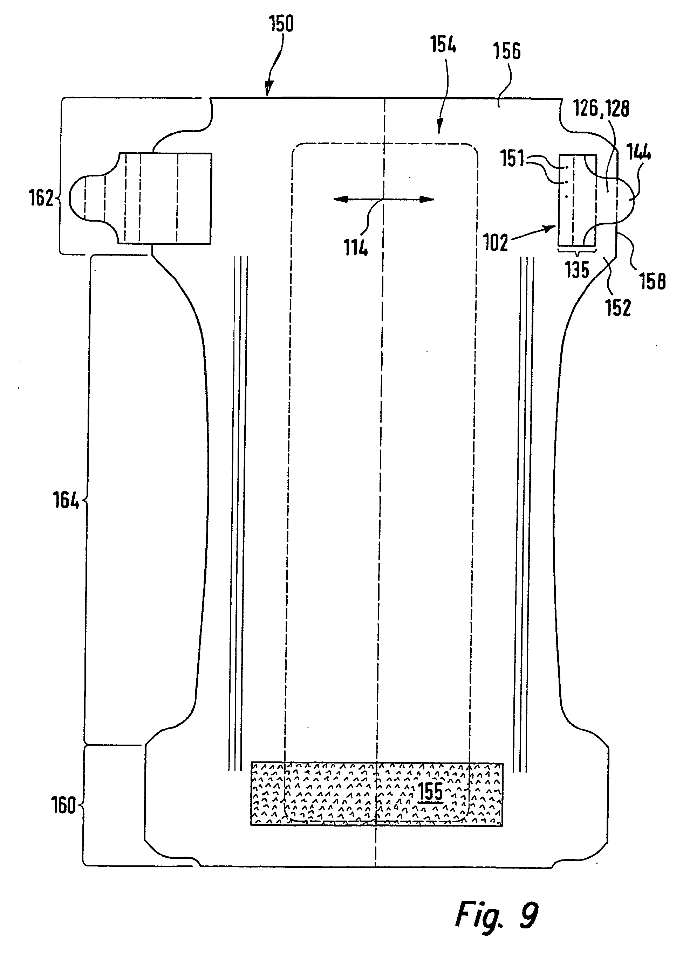

Wenn das Befestigungselement 2 mit seinem Haftkleberauftrag 124 im ersten Bereich 122 auf einen Hygieneartikel aufgebracht ist, so ist - wie bereits erwähnt - der zweite Bereich 126 mit den mechanisch wirkenden Fixiermitteln 127, 128 außerhalb der Z-förmig gefalteten Konfiguration 35 angeordnet und kann somit zum Festlegen des Befestigungselements bzw. des zweiten Abschnitts 108 auf einer textilen Oberfläche eines Hygieneartikels dienen. Dies wird nachfolgend unter Bezugnahme aus Figur 9 erläutert.

Dieses Festlegen des zweiten Abschnitts 108 an dem Hygieneartikel soll aber lediglich der Fixierung des Befestigungselements während der Herstellung und Verpackung bis zum Anlegen des Hygieneartikels an einen Benutzer dienen, zu welchem Zeitpunkt spätestens diese Verbindung gelöst wird. Ein Benutzer greift dann mit seinen Fingern zwischen die Oberseite des Hygieneartikels und einen freien Endbereich 142 des zweiten Abschnitts 108, der dann als Anfassbereich 144 dient.

Figur 9 zeigt eine Draufsicht auf eine erfindungsgemäße Windel 150 mit in einem Rückbereich der Windel 150 beidseits angeordneten Befestigungselementen 102, wie sie im Zusammenhang mit den Figuren 6 bis 8 beschrieben wurden. Auf der in Figur 9 linken Seite ist das Befestigungselement 102 entfaltet und auf der rechten Seite ist es Z-förmig gefaltet dargestellt. Das jeweilige Befestigungselement 102 ist mit seinem ersten Bereich 122 und der dort vorgesehenen rechteckförmigen Kleberbeschichtung 124 auf eine Windelaußenseite, d. h. auf eine beim bestimmungsgemäßen Gebrauch körperabgewandte Oberseite 154 einer flüssigkeitsundurchlässigen Rückschicht 156 der Windel unlösbar aufgebracht. Die Oberseite 154 ist von einer textil anmutenden Vliesbeschichtung einer flüssigkeitsundurchlässigen Folie gebildet. Die Rückschicht 156 ist demzufolge von einem Vlies/Folien-Laminat gebildet.

Das Befestigungselement 102 ist in Querrichtung 114 in einem solchen Abstand von einem Längsrand 158 der Windel 150 in dem Seitenklappen- oder Ohrbereich der Windel 150 angeordnet, dass sie nur mit ihrem Anfassbereich 144 über den Längsrand 150 in Querrichtung 114 vorstehen. Im Besonderen ist dafür Sorge getragen, dass der zweite Bereich 126, in dem die mechanisch wirkenden Verschlusselemente 128 vorgesehen sind, (in Z-förmiger Faltung des Befestigungselements) nicht oder nur sehr wenig, höchstens 5 mm, über den Längsrand 158 in Querrichtung 114 vorstehen. Hierdurch wird verhindert, dass die Verschlusselemente 128 beim Einschlagen der Seitenklappen- oder Ohrbereiche auf die körperzugewandte Deckschichtlage dort festhaken und damit verbunden Beschädigungen auftreten.

Wie aus Figur 9 im Zusammenhang mit Figur 8 ersichtlich ist, befindet sich der zweite Bereich 126 mit den Verschlusselementen 128 außerhalb der Z-förmig gefalteten Konfiguration 135 und liegt somit gegen die Oberseite 154 der Rückschicht 156 der Windel 150 an. Hierbei gehen die mechanisch wirkenden Verschlusselemente 128 eine lösbar haftende Verbindung mit der textil anmutenden Oberseite 154 ein und halten das Befestigungselement 102 in seiner Z-förmig gefalteten Konfiguration 135. Insbesondere wird verhindert, dass sich der zweite Abschnitt 108 mit dem Bereich 126 von der Oberseite 154 löst. Auf diese Weise ist eine Verbesserung der Handhabbarkeit des Hygieneartikels nach dem Anfügen der Befestigungselemente 102 erreicht, und diese bleiben in ihrer Montageposition. Die Windel 150 kann in der Herstellungsmaschine gehandhabt, insbesondere gefaltet, gestapelt und einer Verpackung zugeführt werden.

In Figur 9 links ist der entfaltete Zustand der Befestigungselemente 102 dargestellt. Hierfür ergreift ein Benutzer den Anfassbereich 144 und mit der anderen Hand umfasst er einen Längsrandbereich 152 der Windel 150 und zieht die beiden Komponenten auseinander. Hierdurch wird die lösbar haftende Verbindung der Verschlusselemente 128 an der textil anmutenden Oberseite 154 gelöst, und die Befestigungselemente 102 werden unter Entfaltung der Z-förmig gefalteten Konfiguration 135 in die in Figur 9 links dargestellte Gestalt gebracht. Die Befestigungselemente können nun zum Anlegen der Windel 150 an einen Benutzer auf einen Auftreffbereich 155 in Form eines Schlaufenmaterials im Vorderbereich der Windel 150 gebracht und dort befestigt werden.

Zur weiteren Fixierung der Befestigungselemente 102 in Z-förmig gefalteter Konfiguration 135 ist es denkbar, diskrete Fixierpunkte, etwa Schweißpunkte 151 vorzusehen, welche die Z-förmig übereinander gefalteten Lagen des Befestigungselements 2 lösbar miteinander verbinden.

Man erkennt aus Figur 9 ferner einen Vorderbereich 160, einen Rückenbereich 162 und einen dazwischen liegenden Schrittbereich 164 der Windel 150.

## Patentansprüche

1. Verfahren zum Applizieren von Flachmaterialbahnabschnitten (28) auf eine sich in einer Maschinenrichtung mit einer ersten Bahngeschwindigkeit bewegende erste Flachmaterialbahn (6) im Zuge der Herstellung von Hygieneartikeln oder medizinischen Artikeln, wobei die Abmessung der Flachmaterialbahnabschnitte (28) in der Maschinenrichtung geringer ist als die Abmessung der herzustellenden Artikel, wobei die Flachmaterialbahnabschnitte (28) von einer Endlosbahn (8) im "Cut-and-Place"-Verfahren abgetrennt und auf die erste Flachmaterialbahn (6) aufgebracht werden, und wobei die Endlosbahn (8) mit einer zweiten Bahngeschwindigkeit in Richtung auf eine Messerwalze (18) einer Schneidstation zugeführt wird, **dadurch gekennzeichnet, dass** ein vorderer Abschnitt (26) der Endlosbahn (8) gegen einen Oberflächenabschnitt (24) einer mit der Messerwalze (18) zusammenwirkenden Ambosswalze (22) angelegt wird, der weniger gekrümmt ist als der Umkreis (34) der Ambosswalze (22), und dass der vordere Abschnitt (26) zur Bildung des Flachmaterialbahnabschnitts (28) von der Endlosbahn (8) abgelängt wird und ein jeweiliger abgelängter Flachmaterialbahnabschnitt (28) schlupffrei durch die Ambosswalze (22) beschleunigt wird.

2. Verfahren nach Anspruch 1, **dadurch gekennzeichnet, dass** der vordere Abschnitt (26) der Endlosbahn (8) gegen einen Oberflächenabschnitt (24) der Ambosswalze (22) angelegt wird, dessen Krümmung zylindrisch ist mit einem Krümmungsradius, der größer ist als der Radius des Umkreises (34) der Ambosswalze (22).

3. Verfahren nach Anspruch 1 oder 2, **dadurch gekennzeichnet, dass** der Flachmaterialbahnabschnitt (28) im wesentlichen mit der ersten Bahngeschwindigkeit auf die erste Flachmaterialbahn (6) appliziert wird.

4. Verfahren nach Anspruch 1, 2 oder 3, **dadurch gekennzeichnet, dass** der vordere Abschnitt (26) der Endlosbahn (8) gegen den Oberflächenabschnitt (24) der Ambosswalze (22) durch Unterdruck angesaugt wird.

5. Verfahren nach einem oder mehreren der vorstehenden Ansprüche, **dadurch gekennzeichnet, dass** die Winkelgeschwindigkeit der Ambosswalze (22) periodisch veränderlich gesteuert wird.

6. Verfahren nach Anspruch 5, **dadurch gekennzeichnet, dass** die Winkelgeschwindigkeit der Ambosswalze (22) oder einer weiteren Transportwalze beim Applizieren des Flachmaterialbahnabschnitts (28) auf die erste Flachmaterialbahn (6) derart gesteuert wird, dass die Geschwindigkeit des Flachmaterialbahnabschnitts (28) beim Applizieren der ersten Bahngeschwindigkeit entspricht.

7. Verfahren nach Anspruch 5 oder 6, **dadurch gekennzeichnet, dass** die Winkelgeschwindigkeit der Ambosswalze (22) während des Aufnehmens und Ablängens des Flachmaterialbahnabschnitts (28) derart gesteuert wird, dass die Geschwindigkeit des Flachmaterialbahnabschnitts (28) der zweiten Bahngeschwindigkeit entspricht.

8. Verfahren nach einem oder mehreren der vorstehenden Ansprüche, **dadurch gekennzeichnet, dass** beim Applizieren des Flachmaterialbahnabschnitts (28) auf die erste Flachmaterialbahn (6) eine Andruckwalze (32) auf der von der Ambosswalze (22) abgewandten Seite der ersten Flachmaterialbahn (6) verwendet wird.

9. Verfahren nach einem oder mehreren der vorstehenden Ansprüche, **dadurch gekennzeichnet, dass** eine Endlosbahn (8) zugeführt wird, die um in ihrer Längsrichtung verlaufende Achsen (48, 50, 132, 134) gefaltet, inbesondere z-förmig gefaltet ist.

10. Verfahren nach einem oder mehreren der vorstehenden Ansprüche, **dadurch gekennzeichnet, dass** die aufeinandergefalteten Bahnabschnitte durch Schweiß- oder Klebe- oder Perforationsstellen (151) lösbar aneinander gehalten werden.

11. Vorrichtung (2) zum Applizieren von Flachmaterialbahnabschnitten(28) auf eine sich in einer Maschinenrichtung mit einer ersten Bahngeschwindigkeit bewegende erste Flachmaterialbahn (6) im Zuge der Herstellung von Hygieneartikeln oder medizinischen Artikeln, wobei die Abmessung der Flachmaterialbahnabschnitte (28) in der Maschinenrichtung geringer ist als die Abmessung der herzustellenden Artikel und wobei die Flachmaterialbahnabschnitte (28) von einer Endlosbahn (8) im "Cut-and-Place"-Verfahren abgetrennt und auf die erste Flachmaterialbahn (6) in der Maschinenrichtung aufgebracht werden, mit einer Messerwalze (18) und einer mit dieser zusammenwirkenden Ambosswalze (22), **dadurch gekennzeichnet, dass** die Ambosswalze (22) einen mit dem abzutrennenden Flachmaterialbahnabschnitt (28) zusammenwirkenden Oberflächenabschnitt (24) aufweist, der weniger gekrümmt ist als der Umkreis (34) der Ambosswalze (22).

12. Vorrichtung nach Anspruch 11, **dadurch gekennzeichnet, dass** der Oberflächenabschnitt (24) zylindrisch gekrümmt ist.

13. Vorrichtung nach Anspruch 12, **dadurch gekennzeichnet, dass** der Krümmungsradius des Oberflächenabschnitts (24) 50 -250 mm, insbesondere 65 - 200 mm, insbesondere 80 - 150 mm, insbesondere 90 - 120 mm beträgt.

14. Vorrichtung nach Anspruch 12 oder 13, **dadurch gekennzeichnet, dass** der Krümmungsradius des Oberflächenabschnitts (24) wenigstens das 1,5-fache, insbesondere wenigstens das 1,7-fache, insbesondere wenigstens das 1,8-fache, insbesondere wenigstens das 1,9-fache, insbesondere wenigstens das 2-fache des Radius des Umkreises (34) der Ambosswalze (22) beträgt.

15. Vorrichtung nach einem oder mehreren der Ansprüche 10-14, **dadurch gekennzeichnet, dass** der Radius des Umkreises (34) der Ambosswalze (22) 25 - 75 mm, insbesondere 35 - 65 mm, insbesondere 42 - 52 mm beträgt.

16. Vorrichtung nach einem oder mehreren der Ansprüche 10-15, **dadurch gekennzeichnet, dass** die Messerwalze (18) an ihrem Umfang wenigstens zwei, vorzugsweise wenigstens drei Messer (20) trägt.

17. Vorrichtung nach einem oder mehreren der Ansprüche 10-16, **dadurch gekennzeichnet, dass** die Messer (20) an der Messerwalze (18) federnd nachgiebig gehalten sind.

18. Vorrichtung nach einem oder mehreren der Ansprüche 10-17, **dadurch gekennzeichnet, dass** die Ambosswalze (22) einen einzigen Oberflächenabschnitt (24) zur Aufnahme des Flachmaterialbahnabschnitts (28) aufweist.

19. Vorrichtung nach einem oder mehreren der Ansprüche 10-18, **gekennzeichnet durch** eine Antriebssteuereinrichtung, mittels derer die Winkelgeschwindigkeit der Messerwalze (18) und der Ambosswalze (22) periodisch veränderbar sind.

20. Vorrichtung nach einem oder mehreren der Ansprüche 10-19, die so ausgebildet ist, dass die erste Bahngeschwindigkeit mindestens 50 m/min - 400 m/min beträgt.

21. Vorrichtung nach einem oder mehreren der Ansprüche 10-20, die so ausgebildet ist, dass die zweite Bahngeschwindigkeit 5 - 80 m/min beträgt.

22. Vorrichtung nach einem oder mehreren der Ansprüche 10-21, die so ausgebildet ist, dass die Länge des herzustellenden Artikels in der Maschinenrichtung 30 - 150 cm, insbesondere 45 - 110 cm beträgt.

23. Vorrichtung nach einem oder mehreren der Ansprüche 10-22, die so ausgebildet ist, dass die Abschnittslänge des Flachmaterialbahnabschnitts (28) 1 - 10 cm, insbesondere 3 - 8 cm beträgt.

24. Vorrichtung nach einem oder mehreren der Ansprüche 10-23, die so ausgebildet ist, dass die zuzuführende Endlosbahn (8) um in ihrer Längsrichtung verlaufende Achsen (48, 50, 132, 134) gefaltet, insbesondere z-förmig gefaltet ist.

25. Vorrichtung nach einem oder mehreren der Ansprüche 10-24, **dadurch gekennzeichnet, dass** das Trägheitsmoment der Ambosswalze geringer als 0,0030 kg m², insbesondere geringer als 0,0025 kg m² ist.

26. Vorrichtung nach einem oder mehreren der Ansprüche 10-25, **dadurch gekennzeichnet, dass** das Trägheitsmoment der Messerwalze geringer als 0,0020 kg m², insbesondere geringer als 0,0016 kg m² ist.

27. Vorrichtung nach einem oder mehreren der Ansprüche 10-26, **dadurch gekennzeichnet, dass** das Trägheitsmoment der Andruckwalze geringer als 0,0020 kg m², insbesondere geringer als 0,0016 kg m² ist.

## Claims

1. Method for applying flat material web portions (28) to a first flat material web (6) which moves in a machine direction at a first web velocity during production of hygiene articles or medical articles, the dimensions of the flat material web portions (28) being smaller in the direction of the machine than the dimensions of the articles to be produced, the flat material web portions (28) being separated from an endless web (8) in the cut-and-place method and attached to the first flat material web (6) and the endless web (8) being fed at a second web velocity to a cutting station in the direction of a cutting roller (18), **characterised in that** a front portion (26) of the endless web (8) is applied against a surface portion (24) of an anvil roller (22) which cooperates with the cutting roller (18), said surface portion being less curved than the circumference (34) of the anvil roller (22), and **in that** the front portion (26) is cut from the endless web (8) to form the flat material web portion (28) and each cut flat material web portion (28) is accelerated without slippage by the anvil roller (22).

2. Method according to claim 1, **characterised in that** the front portion (26) of the endless web (8) is applied against a surface portion (24) of the anvil roller (22), said surface portion having cylindrical curvature and a radius of curvature which is greater than the radius of the circumference (34) of the anvil roller (22).

3. Method according to either claim 1 or claim 2, **characterised in that** the flat material web portion (28) is applied to the first flat material web (6) at substantially the first web velocity.

4. Method according to claim 1, claim 2 or claim 3, **characterised in that** the front portion (26) of the endless web (8) is sucked against the surface portion (24) of the anvil roller (22) by means of negative pressure.

5. Method according to one or more of the preceding claims, **characterised in that** the angular velocity of the anvil roller (22) is controlled in a periodically variable manner.

6. Method according to claim 5, **characterised in that** when the flat material web portion (28) is applied to the first flat material web (6), the angular velocity of the anvil roller (22) or another feed roller is controlled in such a way that the velocity of the flat material web portion (28) corresponds during application to the first web velocity.

7. Method according to either claim 5 or claim 6, **characterised in that** when the flat material web portion (28) is being received and cut, the angular velocity of the anvil roller (22) is controlled in such a way that the velocity of the flat material web portion (28) corresponds to the second web velocity.

8. Method according to one or more of the preceding claims, **characterised in that** when the flat material web portion (28) is applied to the first flat material web (6) a pressure roller (32) is used on the side of the first flat material web (6) remote from the anvil roller (22).

9. Method according to one or more of the preceding claims, **characterised in that** an endless web (8) is supplied, which is folded about axes (48, 50, 132, 134), extending in its longitudinal direction and in particular is folded into a z-shape.

10. Method according to one or more of the preceding claims, **characterised in that** the web portions, are folded one onto another, are held together in a detachable manner by welds, joints or perforations (151).

11. Device (2) for applying flat material web portions (28) to a first flat material web (6) which moves in a machine direction at a first web velocity during production of hygiene articles or medical articles, the dimensions of the flat material web portions (28) being smaller in the direction of the machine than the dimensions of the articles to be produced and the flat material web portions (28) being separated from an endless web (8) in the cut-and-place method and attached to the first flat material web (6) in the direction of the machine, comprising a cutting roller (18) and an anvil roller (22) cooperating therewith, **characterised in that** the anvil roller (22) has a surface portion (24) that cooperates with the flat material web portion (28) to be cut and is less curved than the circumference (34) of the anvil roller (22).

12. Device according to claim 11, **characterised in that** the surface portion (24) is cylindrically curved.

13. Device according to claim 12 , **characterised in that** the radius of curvature of the surface portion (24) is 50 to 250 mm, in particular 65 to 200 mm, in particular 80 to 150 mm, and in particular 90 to 120 mm.

14. Device according to either claim 12 or claim 13, **characterised in that** the radius of curvature of the surface portion (24) is at least 1.5 times, in particular at least 1.7 times, in particular at least 1.8 times, in particular at least 1.9 times, and in particular at least twice the radius of the circumference (34) of the anvil roller (22).

15. Device according to one or more of claims 10 to 14, **characterised in that** the radius of the circumference (34) of the anvil roller (22) is 25 to 75 mm, in particular 35 to 65 mm, and in particular 42 to 52 mm.

16. Device according to one or more of claims 10 to 15, **characterised in that** the cutting roller (18) carries at least two, preferably at least three blades (20) on its circumference.

17. Device according to one or more of claims 10 to 16, **characterised in that** the blades (20) are held on the cutting roller (18) so as to be resilient.

18. Device according to one or more of claims 10 to 17, **characterised in that** the anvil roller (22) comprises a single surface portion (24) for receiving the flat material web portion (28).

19. Device according to one or more of claims 10 to 18, **characterised by** a drive control means, by means of which the angular velocities of the cutting roller (18) and the anvil roller (22) are periodically variable.

20. Device according to one or more of claims 10 to 19, which is configured in such a way that the first web velocity is at least 50 m/min to 400 m/min.

21. Device according to one or more of claims 10 to 20, which is configured in such a way that the second web velocity is 5 to 80 m/min.

22. Device according to one or more of claims 10 to 21, which is configured in such a way that the length of the article to be produced is 30 to 150 cm, in particular 45 to 110 cm, in the direction of the machine.

23. Device according to one or more of claims 10 to 22, which is configured in such a way that the portion length of the flat material web portion (28) is 1 to 10 cm, and in particular 3 to 8 cm.

24. Device according to one or more of claims 10 to 23, which is configured in such a way that the endless web (8) to be supplied is folded about axes (48, 50, 132, 134) extending in its longitudinal direction, and in particular is folded into a z-shape.

25. Device according to one or more of claims 10 to 24, **characterised in that** the moment of inertia of the anvil roller is less than 0.0030 kg m², and in particular less than 0.0025 kg m².

26. Device according to one or more of claims 10 to 25, **characterised in that** the moment of inertia of the cutting roller is less than 0.0020 kg m², and in particular less than 0.0016 kg m².

27. Device according to one or more of claims 10 to 26, **characterised in that** the moment of inertia of the pressure roller is less than 0.0020kg m², and in particular less than 0.0016 kg m².

## Revendications

1. Procédé pour appliquer des segments de bande de matériau plat (28) sur une première bande de matériau plat (6) se déplaçant dans un sens machine à une première vitesse de bande, lors de la fabrication d'articles d'hygiène ou d'articles médicaux, la dimension des segments de bande de matériau plat (28) dans le sens machine étant inférieure à la dimension des articles à fabriquer, les segments de bande de matériau plat (28) étant séparés d'une bande sans fin (8) selon le procédé de découpe et mise en place et appliqués sur la première bande de matériau plat (6), et la bande sans fin (8) étant acheminée à une seconde vitesse de bande en direction d'un cylindre porte-couteaux (18) d'une station de coupe, **caractérisé en ce qu'**un segment avant (26) de la bande sans fin (8) est appliqué contre une partie superficielle (24) d'un cylindre enclume (22) coopérant avec le cylindre porte-couteaux (18), laquelle partie superficielle est moins courbée que la circonférence (34) du cylindre enclume (22), **en ce que** le segment avant (26) est séparé de la bande sans fin (8) afin de former le segment de bande de matériau plat (28) et **en ce que** chaque segment de bande de matériau plat (28) séparé est accéléré sans glissement par le cylindre enclume (22).

2. Procédé selon la revendication 1, **caractérisé en ce que** le segment avant (26) de la bande sans fin (8) est appliqué contre une partie superficielle (24) du cylindre enclume (22) dont la courbure est cylindrique et présente un rayon de courbure supérieur au rayon du cercle (34) décrit par le cylindre enclume (22).

3. Procédé selon la revendication 1 ou 2, **caractérisé en ce que** le segment de bande de matériau plat (28) est appliqué essentiellement à la première vitesse de bande sur la première bande de matériau plat (6).

4. Procédé selon la revendication 1, 2 ou 3, **caractérisé en ce que** le segment avant (26) de la bande sans fin (8) est aspiré par dépression contre la partie superficielle (24) du cylindre enclume (22).

5. Procédé selon l'une ou plusieurs des revendications précédentes, **caractérisé en ce que** la vitesse angulaire du cylindre enclume (22) est commandée de manière variable périodiquement.

6. Procédé selon la revendication 5, **caractérisé en ce que** la vitesse angulaire du cylindre enclume (22) ou d'un autre cylindre de transport lors de l'application du segment de bande de matériau plat (28) sur la première bande de matériau plat (6) est commandée de telle manière que la vitesse du segment de bande de matériau plat (28) correspond à la première vitesse de bande lors de l'application.

7. Procédé selon la revendication 5 ou 6, **caractérisé en ce que** la vitesse angulaire du cylindre enclume (22) pendant la réception et la découpe du segment de bande de matériau plat (28) est commandée de telle manière que la vitesse du segment de bande de matériau plat (28) correspond à la seconde vitesse de bande.

8. Procédé selon l'une ou plusieurs des revendications précédentes, **caractérisé en ce que**, lors de l'application du segment de bande de matériau plat (28) sur la première bande de matériau plat (6), on utilise un cylindre de pression (32) sur la face de la première bande de matériau plat (6) orientée à l'opposé du cylindre enclume (22).

9. Procédé selon l'une ou plusieurs des revendications précédentes, **caractérisé en ce qu'**une bande sans fin (8) est acheminée, laquelle est pliée, en particulier pliée en forme de Z, autour d'axes (48, 50, 132, 134) s'étendant dans son sens longitudinal.

10. Procédé selon l'une ou plusieurs des revendications précédentes, **caractérisé en ce que** les segments de bande pliés les uns sur les autres sont tenus entre eux de manière détachable par des points de soudure, de collage ou de perforation (151).

11. Dispositif (2) pour appliquer des segments de bande de matériau plat (28) sur une première bande de matériau plat (6) se déplaçant dans un sens machine à une première vitesse de bande, lors de la fabrication d'articles d'hygiène ou d'articles médicaux, la dimension des segments de bande de matériau plat (28) dans le sens machine étant inférieure à la dimension des articles à fabriquer et les segments de bande de matériau plat (28) étant séparés d'une bande sans fin (8) selon le procédé de découpe et mise en place et appliqués sur la première bande de matériau plat (6) dans le sens machine, comportant un cylindre porte-couteaux (18) et un cylindre enclume (22) coopérant avec celui-ci, **caractérisé en ce que** le cylindre enclume (22) présente une partie superficielle (24) coopérant avec le segment de bande de matériau plat (28) à découper, laquelle partie superficielle est moins courbée que la circonférence (34) du cylindre enclume (22).

12. Dispositif selon la revendication 11, **caractérisé en ce que** la partie superficielle (24) est courbée de manière cylindrique.

13. Dispositif selon la revendication 12, **caractérisé en ce que** le rayon de courbure de la partie superficielle (24) est compris entre 50 et 250 mm, en particulier entre 65 et 200 mm, en particulier entre 80 et 150 mm, en particulier entre 90 et 120 mm.

14. Dispositif selon la revendication 12 ou 13, **caractérisé en ce que** le rayon de courbure de la partie superficielle (24) est égal à au moins 1,5 fois, en particulier au moins 1,7 fois, en particulier au moins 1,8 fois, en particulier au moins 1,9 fois, en particulier au moins 2 fois le rayon du cercle (34) décrit par le cylindre enclume (22).

15. Dispositif selon l'une ou plusieurs des revendications 10 à 14, **caractérisé en ce que** le rayon du cercle (34) décrit par le cylindre enclume (22) est compris entre 25 et 75 mm, en particulier entre 35 et 65 mm, en particulier entre 42 et 52 mm.

16. Dispositif selon l'une ou plusieurs des revendications 10 à 15, **caractérisé en ce que** le cylindre porte-couteaux (18) porte sur son pourtour au moins deux, de préférence au moins trois, couteaux (20).

17. Dispositif selon l'une ou plusieurs des revendications 10 à 16, **caractérisé en ce que** les couteaux (20) sont tenus de manière souple et élastique sur le cylindre porte-couteaux (18).

18. Dispositif selon l'une ou plusieurs des revendications 10 à 17, **caractérisé en ce que** le cylindre enclume (22) présente une seule partie superficielle (24) pour recevoir le segment de bande de matériau plat (28).

19. Dispositif selon l'une ou plusieurs des revendications 10 à 18, **caractérisé par** un dispositif de commande d'entraînement au moyen duquel la vitesse angulaire du cylindre porte-couteaux (18) et du cylindre enclume (22) peut être modifiée périodiquement.

20. Dispositif selon l'une ou plusieurs des revendications 10 à 19, lequel est réalisé de telle manière que la première vitesse de bande est comprise entre au moins 50 m/min et 400 m/min.

21. Dispositif selon l'une ou plusieurs des revendications 10 à 20, lequel est réalisé de telle manière que la seconde vitesse de bande est comprise entre 5 et 80 m/min.

22. Dispositif selon l'une ou plusieurs des revendications 10 à 21, lequel est réalisé de telle manière que la longueur de l'article à fabriquer, dans le sens machine, est comprise entre 30 et 150 cm, en particulier entre 45 et 110 cm.

23. Dispositif selon l'une ou plusieurs des revendications 10 à 22, lequel est réalisé de telle manière que la longueur du segment de bande de matériau plat (28) est comprise entre 1 et 10 cm, en particulier entre 3 et 8 cm.

24. Dispositif selon l'une ou plusieurs des revendications 10 à 23, lequel est réalisé de telle manière que la bande sans fin (8) à acheminer est pliée, en particulier pliée en forme de Z, autour d'axes (48, 50, 132, 134) s'étendant dans son sens longitudinal.

25. Dispositif selon l'une ou plusieurs des revendications 10 à 24, **caractérisé en ce que** le moment d'inertie du cylindre enclume est inférieur à 0,0030 kg m², en particulier inférieur à 0,0025 kg m².

26. Dispositif selon l'une ou plusieurs des revendications 10 à 25, **caractérisé en ce que** le moment d'inertie du cylindre porte-couteaux est inférieur à 0,0020 kg m², en particulier inférieur à 0,0016 kg m².

27. Dispositif selon l'une ou plusieurs des revendications 10 à 26, **caractérisé en ce que** le moment d'inertie du cylindre de pression est inférieur à 0,0020 kg m², en particulier inférieur à 0,0016 kg m².
